# EUROPEAN PATENT APPLICATION

(11) **EP 4 122 477 A1**
(43) Date of publication of application: **25.01.2023**
(21) Application number: 21195897.0
(22) Date of filing: 10.09.2021
(51) Int. Cl.: A61K 35/644, A61P 17/02

(54) **WOUND HONEY WITH A PUMP DISPENSER**

(30) Priority: 21.07.2021 US 202163259509 P; 03.08.2021 US 202163259682 P
(71) Applicant: Fenzl, Mark Edward, Sarasota, FL 34232 (US)
(72) Inventor: Fenzl, Mark Edward, Sarasota, FL 34232 (US)
(74) Representative: Sach, Greg Robert

(57) **Abstract**

An improved wound honey using a combination of buckwheat honey, honeydew honey, a natural thickening agent of blackstrap molasses and tangerine puree, and fragrance comprising lavender essential oil. The wound honey is sterilized using a flowing UV light sanitizing system. The bottles are disinfected using a combination parecetic acid, formic acid, vanillin, clove essential oil, monolaurin, aloe vera, and hydrogen peroxide disinfectant. The product comes in a container with a pump dispenser that dispenses a quantifiable amount of product. This new wound honey with pump dispenser may be used multiple times, is cheaper than manuka honey, and may be produced outside of New Zealand and Australia.

## Description

### CROSS-REFERENCE TO RELATED APPLICATIONS

This application claims the benefit of U.S. Provisional Application 63/259,509, filed on July 21, 2021. The entire disclosure of the above application is incorporated herein by reference.

This application claims the benefit of U.S. Provisional Application 63/259,682, filed on August 3, 2021. The entire disclosure of the above application is incorporated herein by reference.

### FIELD

The present application relates to a natural thickened wound honey that is equivocal to manuka honey in antimicrobial and wound healing effectiveness and has a multiple use pump dispenser that can dispense a quantifiable amount of product. The product is dispensed from a clove-vanilla scented bottle.

### BACKGROUND

The present invention relates to an improved natural thickened wound honey that is equivocal to manuka honey gel in antimicrobial and wound healing effectiveness, but may be made in North America and is cheaper. The wound honey gel is dispensed from a container with a pump dispenser that can provide a quantifiable amount dispensed and dispense multiple times. Manuka Honey comes from Australia and New Zealand because that is where *Leptospermum scoparium* trees grow. It has been studied for its antimicrobial and wound healing properties. Manuka honey contains higher levels of polyphenols than other honeys, which provides a stronger antimicrobial effect. Manuka honey also contains methylglyoxal instead hydrogen peroxide. Most other honeys have hydrogen peroxide, which has antimicrobial effects, but methylglyoxal has a stronger antimicrobial effect. Manuka honeys containing an appropriate amount of methylglyoxal have been approved in the United States to be used as wound honey. Currently, the leading wound honey gel in the United States is a high methylglyoxal manuka honey with twenty percent natural gelling agent to thicken the honey. Problems with the current product are that manuka honey only comes from New Zealand or Australia, is very expensive, and only comes in single use tubes. A problem with honey in general is that it has a tendency to run and adding a thickening agent will increase viscosity and increase adhesion to bandages and wounds. Another problem is that the sugar in honey has a tendency to crystallize when sitting for long periods of time at cool temperatures, giving it a hard, gritty or rock-like consistency. This can be fixed by gentle heating, but this takes time and effort.

North American Buckwheat honey contains higher levels of polyphenols than other honeys and has equivalent antimicrobial efficacy as manuka honey. Honeydew honey has strong antimicrobial efficacy. Blackstrap molasses inhibits crystallization, contains higher levels of antioxidant polyphenols than honey, has an antimicrobial effect, and is more viscous than honey. Tangerine puree inhibits crystallization, has a broad-spectrum antimicrobial effect, and is more viscous than honey. Tangerine puree naturally contains limonene and thymol that provide fragrance and antimicrobial properties. Lavender essential oil has been found to decrease wound healing time and provide a beneficial scent to the patient and providers. Lavender essential oil contains the fragrant antimicrobial chemicals linalool and linalyl acetate.

**Table 1. Viscosity of Ingredients in centipoise**

| Ingredient | Black Strap Molasses | Orange Juice Concentrate | Honey |
|---|---|---|---|
| Viscosity | 5,000 - 10,000 cps | 5,000 cps | 2,000-3,000 cps |

By combining buckwheat honey and honeydew honey with a natural thickening agent containing black strap molasses and tangerine puree and a fragrance associated with decreased wound healing time, I have invented a new natural synergistically antimicrobial thickened wound honey with a pleasant color and scent. The citric acid in tangerine puree has a synergistic antimicrobial effect with the hydrogen peroxide created by buckwheat honey. Lavender essential oil has a synergistic antimicrobial effect with thymol found in tangerine puree.

**Table 2. Synergy of citric acid with hydrogen peroxide**

| *Salmonella sp.* | Minimal Bactericidal Concentration |
|---|---|
| Citric Acid | 5% |
| Hydrogen Peroxide | 3.12% |
| Combination | 0.625% / 1.56% |

**Table 3. Synergy of lavender essential oil with thyme essential oil with 21% thymol**

| | |
|---|---|
| *Staphylococcus aureus* ATCC 6538 | Minimal Inhibitory Concentration |
| *Lavandula angustifolia* Essential Oil | 2 mg / ml |
| *Thymus vulgaris* Essential Oil | 3.33 mg / ml |
| Combination 1:1 | 1 ml |

One example of several potential wound honeys, that does not limit this patent application, is a thickened wound honey comprising approximately more than 4% buckwheat honey, more than 4% honeydew honey, between 4% and 51% black strap molasses, between 1% and 51% citrus puree, and a fragrance comprising between 0.01% and 5% linalool and between 0.01% and 5% linalyl acetate. Percentages are based on the final product. The product is sanitized using a flowing UV light sanitizing system, meaning that the honey is pumped past a UV light that sterilizes the honey. The bottles are disinfected using a combination of between 0.01% and 35% peracetic acid, between 0.01% and 35% acetic acid, between 0.01% and 35% formic acid, between 0.01% and 10% monolaurin, between 0.01% and 10% clove essential oil, between 0.01% and 10% vanillin, between 0.01% and 10% aloe vera powder, between 1% and 20% propylene glycol, and between 1% and 99.9% hydrogen peroxide. Percentages are based on the final disinfectant.

The wound gel uses a pump dispenser so that the amount dispensed can be measured and the bottle may be used multiple times. This helps the medical assistant, nurse, or wound nurse quantify how much wound honey gel is to be applied. This helps the physician set the amount prescribed. This helps the pharmacist, medical supply, insurance, and health care administrators determine how much is being ordered and used. This also helps physicians and researchers determine the most effective dose per surface area. It will save cost for the product and saves cost of waste associated with a single use container. Before the product is applied, three full pumps of the product are placed on disposable guaze, napkin, toilet paper, or paper towel and disposed to clear the pump dispenser of any airborne contaminants prior to use. Then the product is dispensed on the bandage to be applied.

The bottles after disinfection will have a clove-vanilla scent. This provides a pleasant scent for the manufacturers and product workers.

As unlimited examples for this patent, the product may be sold in ½ ounce, 1 ounce, 2 ounce, 4 ounce, 8 ounce, ½ gallon, 1 gallon, 5 gallon, 55 gallon, and 300 gallon containers with an associated pump dispenser. The pump dispenser may be attached to the bottle or may be separate and activated by hand, foot pump, or motorized pump. The container may be transported as a package or on a pallet.

There is public fear of health risk associated with parabens and formaldehyde donor preservatives. This unique wound honey gel would only contain natural occurring products and will not contain parabens or formaldehyde donors.

Three ways a product is protected from microbial spoilage are water activity level, chelation, and acidity. Products with low water activity level do not have enough water for bacteria or fungus reproduce and cause damage. Chelation binds free metals in a solution preventing microbes from using them. Acidity or low pH damage microbes, preventing them from damaging the product. Honey naturally has a low water activity level and pH. By combining ingredients with low water activity, chelating effect, and acidity, such as mixing the citric acid found in tangerine puree with hydrogen peroxide created by buckwheat honey, has a synergistic antimicrobial effect.

### SUMMARY OF THE INVENTION

This invention is an improved natural thickened wound honey that is cheaper, smells better, and with a more attractive appearance than manuka honey gel and can be produced outside of New Zealand and Australia. One example of several potential wound honeys, that does not limit this patent application, is a wound honey gel comprising more than 5% buckwheat honey, more than 5% honeydew honey, between 5% and 50% black strap molasses, between 2% and 50% citrus puree, between 0.01% and 3% limonene, between 0.01% and 3% thymol, between 0.01% and 3% linalyl acetate, and between 0.01% and 3% linalool. The product is sanitized using a flowing UV light sanitizing system. The bottles are disinfected using between 0.01% and 3% peracetic acid, between 0.01% and 3% acetic acid, between 0.01% and 3% formic acid, between 0.01% and 5% monolaurin, and between 0.01% and 5% clove essential oil, between 0.01% and 5% vanillin, between 0.01% and 5% aloe vera powder, between 1% and 15% propylene glycol, and between 3% and 99.9% hydrogen peroxide disinfectant. The wound gel uses a pump applicator to dispense a measured amount of product. The pump is cleared of contaminants by pumping and discarding three full pumps of product before use.

### DETAILED DESCRIPTION

One example of several potential wound honeys, that does not limit this patent application, is a thickened wound honey comprising 50% buckwheat honey, 30% honeydew honey, a thickening agent comprising 15% black strap molasses and 4.9% tangerine puree, a fragrance comprising 0.1% lavender essential oil. The product is sanitized using a flowing UV light sanitizing system. In other words, the liquid product is piped through UV light in order to sanitize the product. The bottles are sanitized using a solution comprising 0.2% parecetic acid, 0.3% acetic acid, 4% formic acid, 2% clove essential oil, 5% vanillin, 1% monolaurin, 0.2% aloe vera powder, 10% propylene glycol, and 79.3% hydrogen peroxide at 33% concentration. The wound gel may use a pump applicator so the medical assistant, nurse, physician, pharmacist, researcher, medical supply, medical insurance, and hospital administration know how much is to be applied and how often. The product may be bottled, in examples that do not limit this patent, in a ½ ounce bottle, a 1 ounce bottle, a 2 ounce bottle, a 4 ounce bottle, an 8 ounce bottle, a ½ gallon bottle, a 1 gallon bottle, a 55 gallon drum, or a 300 gallon container. The containers have a pleasant clove-vanilla scent. Three full pumps of the product are dispensed on a disposable paper and then disposed to clear contamination of the pump before the product is used.

Percentages in this patent refer to final product concentration of the entire product. Fruit concentrate in this patent refers to any dehydrated product including but not limited to frozen fruit concentrate, sterilized fruit concentrate, fruit extract, fruit paste, fruit syrup, or fruit molasses.

Citrus concentrate in this patent refers to any dehydrated product including but not limited to frozen citrus concentrate, sterilized citrus concentrate, citrus extract, citrus paste, citrus syrup, or citrus molasses.

## Claims

1. A wound product comprising at least two of buckwheat honey at a concentration more than 4%, honeydew honey at a concentration more than 4%, molasses at a concentration between 4% and 51%, citrus concentrate at a concentration between 1% and 51%, limonene at a concentration between 0.01% and 5%, thymol at a concentration between 0.01% and 5%, linalyl acetate at a concentration between 0.01% and 5%, and linalool at a concentration between 0.01% and 5%.

2. A product in claim 1 that is dispensed with a pump dispenser.

3. A container associated with a dispenser pump that contains a wound product in claim 1.

4. A container in claim 3 disinfected with at least two of formic acid, acetic acid, eugenol, vanillin, monolaurin, aloe vera, and hydrogen peroxide.

5. A bottle containing a product in claim 1 that has a clove-vanilla scent.

6. A wound dressing that is dispensed through a pump in quantifiable amounts.

7. A dispenser pump or a container with an associated dispenser pump to dispense a wound dressing used in Claim 6.

8. A bottle with a clove-vanilla scent and a dispenser pump that dispenses a wound dressing in claim 6.

9. A wound dressing in Claim 6 that comprises at least one of honey, artificial honey, molasses, syrup, fruit puree, and fruit concentrate.

10. A product in Claim 6 in which the containers are disinfected using at least two of formic acid, acetic acid, eugenol, vanillin, aloe vera, monolaurin, and hydrogen peroxide.

11. A honey product sanitized by flowing through a UV light sanitizing system.

12. A product in claim 11 that is a wound honey.

13. A product in claim 11 in which the product bottles are disinfected with at least two of formic acid, acetic acid, eugenol, vanillin, aloe vera, monolaurin, and hydrogen peroxide.

14. A product in claim 11 that is dispensed from a clove-vanilla scented bottle.

15. A honey product in Claim 11 that comprises at least one of honey, artificial honey, molasses, syrup, fruit concentrate, and fruit puree.
